(19) Europäisches Patentamt
European Patent Office
Office européen des brevets

(11) Veröffentlichungsnummer: **0 034 804**
**B1**

# (12) EUROPÄISCHE PATENTSCHRIFT

(45) Veröffentlichungstag der Patentschrift: **23.03.83**

(51) Int. Cl.³: **C 07 C 45/62**, C 07 C 49/04

(21) Anmeldenummer: **81101164.2**

(22) Anmeldetag: **19.02.81**

## (54) Verfahren zur Herstellung von höheren gesättigten Ketonen.

(30) Priorität: **23.02.80 DE 3006867**

(43) Veröffentlichungstag der Anmeldung: **02.09.81 Patentblatt 81/35**

(45) Bekanntmachung des Hinweises auf die Patenterteilung: **23.03.83 Patentblatt 83/12**

(84) Benannte Vertragsstaaten: **CH DE FR GB LI NL**

(56) Entgegenhaltungen:
**DE-A-2 640 026**
**DE-B-1 196 632**
**FR-A-1 179 925**

(73) Patentinhaber: **BASF Aktiengesellschaft, Carl-Bosch-Strasse 38, D-6700 Ludwigshafen (DE)**

(72) Erfinder: **Arnold, Lothar, Dr., Hausackerweg 11, D-6900 Heidelberg (DE)**
Erfinder: **Dehler, Juergen, Dr., Im Eichenweg 4, D-6701 Neuhofen (DE)**
Erfinder: **Hoffmann, Werner, Dr., Ringstrasse 11 C, D-6701 Neuhofen (DE)**
Erfinder: **Kroesche, Henning, Dr., Lorscher Ring 4 C, D-6710 Frankenthal (DE)**

**EP 0 034 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von höheren gesättigten Ketonen

Die Erfindung betrifft ein verbessertes Verfahren zur industriellen Herstellung von gesättigten terpenoiden Ketonen der allgemeinen Formel I

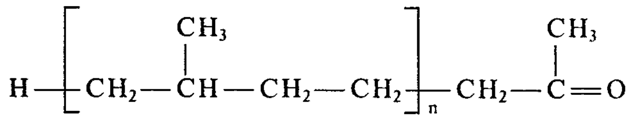

(I)

in der n für 1, 2, 3 oder 4 steht durch katalytische Hydrierung an Pd enthaltenden Katalysatoren, insbesondere von Hexahydrofarnesylaceton (auch als Phyton bekannt).

Die terpenoiden Ketone der Formel I stellen sehr wertvolle chemische Zwischenprodukte für die Synthese zahlteicher Naturstoffe dar. So hat beispielsweise das Hexahydrofarnesylaceton große Bedeutung als Zwischenprodukt für Isophytol und damit für das begehrte Vitamin E. Dementsprechend sind zahlreiche Verfahren zur Herstellung der Ketone der Formel I bekannt, in denen diese letztlich durch Hydrierung der entsprechenden ein- oder mehrfach ungesättigten Ketone hergestellt werden.

So ist z. B. aus J. Agr. Food Chem., Vol. 21 No. 3 (1973) Seite 357 bekannt, Geranylaceton (6.10.-Dimethyl-5.9-un decadien-2-on) durch Hydrieren an Pd/C in 6,10 Dimethyl-undeca-2-on zu überführen.

Aus dem Z. obsc. chim. 32 (1962) Nr. 8, Seiten 2483—2487 ist es bekannt, 2-Methyl-heptanon-(2) durch Hydrieren von Pseudojonon (6.10-Dimethyl-3.5.9-undecatrien-2-on) an Pd/C zu gewinnen.

Aus der US-PS 3 728 395 ist es bekannt, 6,10-Dimethyl-dodecan-2-on durch Hydrieren von 6,10-Dimethyl-dodeca-9-en-2-on an Pd/C zu erhalten.

Aus der US-PS 3 917 710 ist es bekannt, 6,10,14-Trimethyl-pentadecan-2-on durch katalytisches Hydrieren von 6,10-14-Trimethyl-13-pentadecen-2-on in Gegenwart von Pd/C Raney-Ni oder PtO, herzustellen.

Aus DE-OS 2 640 026 ist u. a. die Herstellung von Hexahydrofarnesylaceton durch katalytische Hydrierung der entsprechenden 3-; 3.9- oder 3,9,13-ungesättigten Verbindung bekannt, wobei man als Katalysator vorzugsweise Edelmetalle wie Pd verwendet. Gemäß einem Beispiel wird das 3,13-ungesättigte Keton bei 80° C zwei Stunden an Pd/C hydriert.

In allen Literaturstellen ist angegeben, daß die ungesättigten Ketone in guten bis quantitativen Ausbeuten erhalten werden.

Bei der Einführung der katalytischen Hydrierung in Gegenwart von Palladium auf Aktivkohle in die Synthese in industriellem Maßstab ergeben sich jedoch schwerwiegende Nachteile.

Beispielsweise sind bisher keine Pd-Kohle-Katalysatoren erhältlich, die für eine kontinuierliche Reaktionsführung geeignet wären und die Abtrennung der pulverförmigen Katalysatoren ist technisch recht aufwendig. Weiterhin sind für Hydrierungen, wie der des erfindungsgemäßen Verfahrens, im allgemeinen Katalysatoren notwendig, die das teure Palladium in sehr hohen Konzentrationen auf der Kohle enthalten. Weiterhin gelingt die Hydrierung oft nur gut, wenn man in größeren Mengen eines Lösungsmittels arbeitet, was technisch großen Aufarbeitungsaufwand mit sich bringt.

Es wurde daher versucht, die katalytische Hydrierung der ungesättigten Ketone an solchen Pd-Katalysatoren vorzunehmen, die als Trägermaterial Calciumcarbonat oder Kieselgel enthielten. Versuche im Labormaßstab verliefen erfolgversprechend. Bei Langzeitversuchen mit Palladium-Kieselgel-Katalysatoren im größeren Maßstab zeigten sich jedoch erhebliche Nachteile.

Die Herstellung eines Katalysators mit reproduzierbaren Eigenschaften erwies sich als recht schwierig. Hierdurch bedingt sind sowohl die Chargendauer als auch die Produktqualitäten sehr unterschiedlich, was einen hohen Überwachungsaufwand für die Anlage notwendig macht. Weiterhin traten des öfteren Fehlchargen auf, d. h., daß die Ketone beispielsweise unter Bildung des entsprechenden Alkohols durchhydriert oder zu leichtsiedenden Anteilen aufgespalten wurden. Ein weiterer Nachteil der Palladium-Kieselsäure-Katalysatoren liegt darin, daß sie durch Chlorionen rasch vergiftet und damit irreversibel desaktiviert werden.

Es war daher die Aufgabe der Erfindung, einen Katalysator zu finden, der auf einfache Weise herstellbar und technisch zu handhaben ist und dabei reproduzierbare Eigenschaften aufweist, der weder den Angriff des Wasserstoffs auf die Carbonylgruppe noch die Spaltung des ungesättigten Ketons katalysiert, der relativ unanfällig gegen Kontaktgifte, wie Chlorionen ist und leicht vom Reaktionsprodukt abgetrennt werden kann.

Es wurde nun überraschenderweise gefunden, daß Katalysatoren die Pd auf Aluminiumoxid als Trägermaterial enthalten, für die Herstellung der Ketone der Formel I aus den entsprechenden ungesättigten Ketonen hervorragend geeignet sind und die Nachteile der Katalysatoren nach dem Stande der Technik nicht aufweisen. Überraschend war die Tatsache u. a. auch deshalb, weil es bekannt war, daß man Dehydrolinalool (III) an $Pd/Al_2O_3$-Kontakten mit hoher Selektivität (Ausbeute etwa 99%) zu dem zweifach olefinisch ungesättigten Linalool (IV) partiell hydrieren kann.

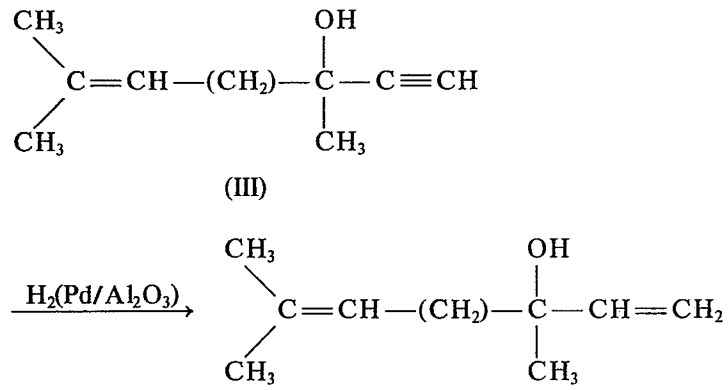


(III)

(IV)

Gegenstand der Erfindung ist dementsprechend ein Verfahren zur Herstellung von Ketonen der allgemeinen Formel I

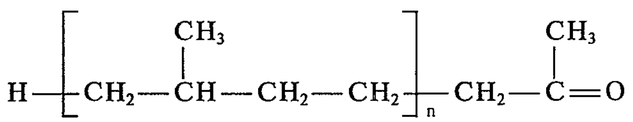

(I)

in der n für 1, 2, 3 oder 4 steht, durch katalytische Hydrierung der entsprechenden einfach oder mehrfach ungesättigten Ketone in Gegenwart von Palladium enthaltenden Katalysatoren, das dadurch gekennzeichnet ist, daß man die Hydrierung in Gegenwart von Katalysatoren durchführt, die 0,05 bis 5 Gew.-% vorzugsweise 0,1 bis 3, insbesondere 0,2 bis 0,8 Gew.-% Palladium auf Aluminiumoxid als Trägermaterial enthalten.

Die erfindungsgemäß hydrierbaren ungesättigten Ketone können etwa auf die folgende Weise dargestellt werden:

Ketone der allgemeinen Formel II

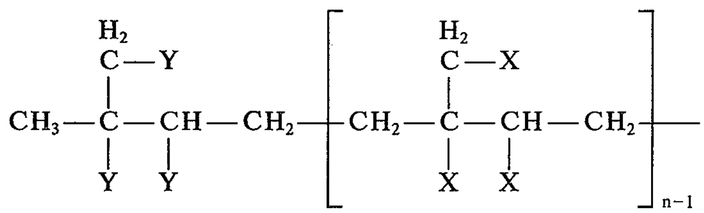

(II)

in der jeweils 2 benachbarte Y für eine zusätzliche Bindung zwischen den diese Y tragenden C-Atomen bedeuten, während das dritte Y für H steht und die 3 X entweder für H stehen oder aber je 2 benachbarte X zusammen für eine zusätzliche Bindung zwischen den sie tragenden C-Atomen stehen und das dritte X für H steht.

Besondere Bedeutung hat das Verfahren für Ketone der allgemeinen Formel IIa

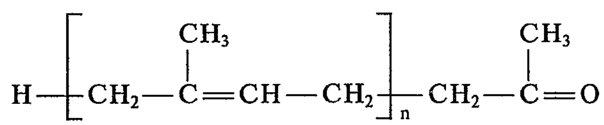

(IIa)

unter die beispielsweise das Geranylaceton (n = 2) und insbesondere das Farnesylaceton (n = 3) fallen.

Als mögliche Ausgangsketone seien weiterhin genannt:

6-Methyl-hepten-5-on-2, 6-Methylhepten-6-on-2, 6,10-Dimethyl-undeca-5-en-2-on, 6,10,14-Trimethyl-pentadeca-5,9-dien-2-on, 6,10,14-Trimethyl-pentadeca-5-en-2-on und 6,10,14,18-Tetramethyl-nonadeca-5,9,13,17-tetra-en-2-on.

Die $Pd/Al_2O_3$-Katalysatoren können in Pulverform verwendet werden. Bevorzugt wird jedoch die Verwendung von geformten Katalysatorpartikeln, beispielsweise ein Katalysator in Form von Granulat oder in Form von Strängen.

Ein besonderer Vorteil der erfindungsgemäßen Katalysatoren ist, daß sie bei eventuellem Nachlassen der Aktivität auf einfache Weise durch Behandeln mit Luftsauerstoff bei Temperaturen von 20 bis 150°C reaktiviert werden können.

Die Reaktionstemperatur für die erfindungsgemäße Hydrierung beträgt im allgemeinen 50 bis 150° C, vorzugsweise 70 bis 110° C; der benötigte Wasserstoffdruck im allgemeinen etwa 0 bis 20 bar, vorzugsweise 1 bis 10 bar.

Die Mitverwendung von inerten Lösungsmitteln ist zwar prinzipiell möglich, ist jedoch im allgemeinen nicht erforderlich.

Die Hydrierung kann diskontinuierlich durchgeführt werden, jedoch wird die kontinuierliche Hydrierung besonders bevorzugt.

Die Reaktionszeit liegt im allgemeinen zwischen 3 und 30, insbesondere zwischen 3 und 15 Stunden.

Mit Hilfe des erfindungsgemäßen Verfahrens ist es möglich, die als Zwischenprodukte für zahlreiche terpenoiden Naturstoffe begehrten Ketone der Formel I durch katalytische Hydrierung entsprechender einfach oder mehrfach olefinisch ungesättigter Ketone in industriellem Maßstab auf sehr vorteilhafte Weise herzustellen. Während der 3jährigen Erprobung des Verfahrens im Technikumsmaßstab traten keine Fehlchargen auf, sondern es wurde ein Produkt gleichbleibender Qualität erzielt. Der Katalysator konnte jeweils auf einfachste Weise wieder reaktiviert werden.

## Beispiel 1

Als Reaktionsgefäß diente ein mit Mantelheizung bzw. -kühlung ausgestattetes Hochdruckrohr (Nennweite 50 mm und 3 l Inhalt), das mit einem Palladium/Aluminiumoxid-Katalysator gefüllt war. Der Katalysator war zylinderförmig: die Stranglänge lag zwischen 4 und 12 mm, der Durchmesser zwischen 3 und 8 mm. Der Palladiumgehalt betrug ca. 0,5%.

In diesen Reaktor wurden stündlich 200 ml (= 175 g) 6.10.14-Trimethyl-pentadeca-5.9.13-trien-2-on (Farnesylaceton) gepumpt. Wasserstoff wurde so zudosiert, daß am Reaktorausgang der Druck 5 bar betrug. Die Reaktionstemperatur wurde mit Hilfe eines Mischwasserkreises auf 80 bis 100° C über den Reaktor verteilt eingestellt. Es wurden stündlich 210—215 ml Reaktionsprodukt erhalten.

Die Reaktion wurde mit Hilfe der Gaschromatographie und titrimetrischer Bromzahlbestimmung überwacht. Die Bromzahl muß unter 5 liegen.

Bei der fraktionierten Destillation des stündlich anfallenden Produktes erhielt man im Mittel 169 g Hauptlauf, der nach GC-Analyse 98%iges Hexahydrofarnesylaceton war (Ausbeute 93%). Kp = 127° C bei 0,07 mbar; $n_D^{25}$ = 1.4441.

Die Umsetzung wurde in diesem System 9 Monate störungsfrei betrieben. Danach stieg die Bromzahl über 5 und der Katalysator wurde wie folgt reaktiviert:

Zulauf und Wasserstoffzufuhr wurden abgestellt und das Reaktionsgefäß 24 Stunden mit 5 l Stickstoff pro Stunde gespült. Dann wurden bei 80—100° C stündlich 10 l Luft durchgefahren.

Nach 5 Stunden wurde die Luft, wie oben geschildert, mit Stickstoff verdrängt. Anschließend wurde Hydrierung wie oben beschrieben fortgesetzt.

Diese Prozedur wurde jeweils nach 9 Monaten noch zweimal wiederholt; ein Nachlassen der Katalysatoraktivität war auch danach noch nicht festzustellen.

Unter gleichen Bedingungen und mit ähnlichen Ergebnissen wurden hydriert:

6.10.14-Trimethyl-pentadeca-5.9-dien-2-on zu Hexahydrofarnesylaceton.

6.10-Dimethyl-undeca-5.9-dien (Geranylaceton) zu Tetrahydrogeranylaceton Kp = 75°C bei 0,07 mbar; $n_D^{25}$ = 1,4328.

2.6-Dimethyl-hept-5-en-2-on (Methylhepton) zu Methylheptanon Kp = 67°C bei 26,6 mbar; $n_D^{25}$ = 1,4162.

## Beispiel 2

### A Erfindungsgemäße Umsetzung

131 g (0,5 Mol) Farnesylaceton (nach gaschromatographischer Untersuchung 99,7%ig rein; Struktur durch ein NMR-Spektrum belegt; Schwefel- und Halogen-frei wurden mit 1 g eines pulverförmigen Palladium/$Al_2O_3$-Katalysators (0,5%ig) versetzt. In das Reaktionsgemisch wurde bei 90° C unter Rühren Wasserstoff eingeleitet. Stündlich wurden Proben gaschromatographisch untersucht. Nach 20 Stunden war die Hydrierung von Farnesylaceton zu Hexahydrofarnesylaceton quantitativ beendet. Der Katalysator wurde abfiltriert und das Filtrat destillativ aufgearbeitet. Man erhielt das Hexahydrofarnesylaceton in 98%iger Reinheit und mit einer Ausbeute von 95%.

### B Vergleichsversuche

a) 131 g (0,5 Mol) des oben beschriebenen Farnesylacetons wurden mit 1 g eines Palladium/Aktivkohlekatalysators (1%ig) versetzt und in das Reaktionsgemisch unter Rühren Wasserstoff

eingeleitet. Weder bei 20°C, noch bei 50°C oder 90°C konnte eine Wasserstoffaufnahme beobachtet werden.

b) Arbeitete man wie unter a) beschrieben, verwendete jedoch anstelle von 1 g eines 1%igen Palladium/Aktivkohlekatalysators 1 g eines 2%igen Pd/C-Katalysators, so konnte ebenfalls keine Wasserstoffaufnahme beobachtet werden.

c) 131 g (0,5 Mol) des oben beschriebenen Farnesylacetons wurden mit 1 g eines 5%igen Pd/C-Katalysators versetzt und in das Reaktionsgemisch unter Rühren $H_2$ eingeleitet. Bei 20°C wurden innerhalb von 24 Stunden 16 Liter $H_2$ aufgenommen, anschließend bei 50°C in 8 Stunden nochmals 5 Liter $H_2$ und danach bei 90°C in 30 Stunden nochmals 11,6 Liter. Insgesamt wurden also in 64 Stunden 32,6 Liter $H_2$ (Theorie: 38 Liter $H_2$) aufgenommen. Das Reaktionsprodukt enthielt nach gaschromatographischer (GC) Analyse etwa 67% Hexahydrofarnesylaceton. Nach NMR-spektroskopischen Untersuchungen war in dem Produkt im wesentlichen nur noch die der Carbonylgruppe am nächsten stehende Doppelbindung nachweisbar.

d) 131 g (0,5 Mol) des oben beschriebenen Farnesylacetons wurden mit 1 g eines 5%igen Pd/C-Katalysators versetzt und in das Reaktionsgemisch bei 90°C unter Rühren $H_2$ eingeleitet. Nach 30 Stunden war die $H_2$-Aufnahme beendet. Das Reaktionsprodukt enthielt nach GC-Analyse nur etwa 68% an dem gewünschten Hexahydrofarnesylaceton.

e) 131 g des oben beschriebenen Farnesylacetons wurden mit 1 g eines 10%igen Pd/C-Katalysators versetzt und in das Reaktionsgemisch bei 90°C unter Rühren $H_2$ eingeleitet. Nach 32 Stunden war die $H_2$-Aufnahme beendet. Das Reaktionsprodukt enthielt nach GC-Analyse nur etwa 49% des gewünschten Hexahydrofarnesylacetons.

## Beispiel 3

### A Erfindungsgemäße Umsetzung

131 g (0,5) des oben beschriebenen Farnesylacetons wurden mit 1 g eines pulverförmigen Pd/$Al_2O_3$-Katalysators (0,5%ig) versetzt. Das Reaktionsgemisch wurde in einem Autoklaven unter einem $H_2$-Druck von 5 bar auf 90°C erhitzt. Nach 8 Stunden war die Hydrierung vollständig. Der Katalysator wurde abfiltriert und das Filtrat destillativ aufgearbeitet. Man erhielt das gewünschte Hexahydrofarnesylaceton in 98%iger Reinheit und mit einer Ausbeute von 95% der Theorie.

### B Vergleichsversuch

131 g des oben beschriebenen Farnesylacetons wurden mit 1 g eines 10%igen Pd/C-Katalysators versetzt. Das Reaktionsgemisch wurde in einem Autoklaven unter einem $H_2$-Druck von 5 bar auf 90°C erhitzt. Nach beendeter $H_2$-Aufnahme waren im Reaktionsprodukt nur etwa 70% Hexahydrofarnesylaceton neben nicht vollständig hydrierten Verbindungen enthalten.

**Patentansprüche**

1. Verfahren zur Herstellung von Ketonen der allgemeinen Formel I

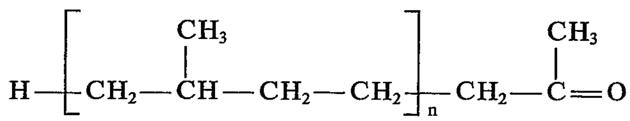

(I)

in der n für 1, 2 3 oder 4 steht, durch katalytische Hydrierung der entsprechenden einfach oder mehrfach ungesättigten Ketone in Gegenwart von Palladium enthaltenden Katalysatoren, dadurch gekennzeichnet, daß man die katalytische Hydrierung in Gegenwart von Katalysatoren durchführt, die 0,05 bis 5 Gew.-% Palladium auf Aluminiumoxid als Trägermaterial enthalten.

2. Verfahren zur Herstellung von Ketonen der allgemeinen Formel I gemäß Anspruch 1, dadurch gekennzeichnet, daß man die Hydrierung in Gegenwart von Katalysatoren durchführt, die 0,2 bis 0,8 Gew.-% Palladium auf Aluminiumoxid enthalten.

**Claims**

1. A process for the preparation of a ketone of the general formula I

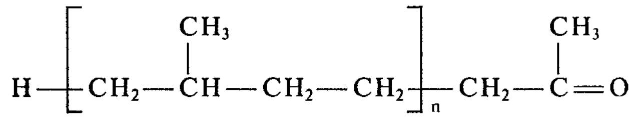

(I)

where n is 1, 2, 3 or 4, by catalytically hydrogenating a corresponding mono-unsaturated or poly-unsaturated ketone over a palladium-containing catalyst, wherein the catalytic hydrogenation is carried out over a catalyst which contains from 0.05 to 5% by weight of palladium on aluminium oxide as the carrier.

2. A process for the preparation of a ketone of the general formula I as claimed in claim 1, wherein the hydrogenation is carried out over a catalyst which contains from 0.2 to 0.8% by weight of palladium on aluminium oxide.

**Revendications**

1. Procédé pour la production de cétones de formule générale

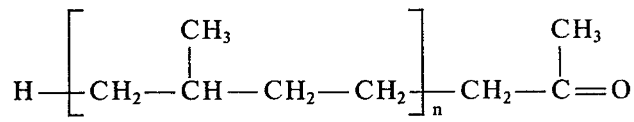

(I)

dans laquelle n est mis pour 1, 2, 3 ou 4, par hydrogénation catalytique des cétones à insaturation simple ou multiple correspondantes en présence de catalyseurs contenant du palladium, caractérisé en ce qu'on effectue l'hydrogénation catalytique en présence de catalyseurs qui contiennent 0,05 à 5% en poids de palladium sur un oxyde d'aluminium servant de matière de support.

2. Procédé pour la production de cétones de formule générale I, caractérisé en ce qu'on effectue l'hydrogénation en présence de catalyseurs qui contiennent 0,2 à 0,8% en poids de palladium sur un oxyde d'aluminium.